(19) Europäisches Patentamt
European Patent Office
Office européen des brevets

(11) EP 2 473 849 B1

(12) **EUROPEAN PATENT SPECIFICATION**

(45) Date of publication and mention of the grant of the patent:
**29.10.2014 Bulletin 2014/44**

(21) Application number: **10774286.8**

(22) Date of filing: **02.09.2010**

(51) Int Cl.:
***G01N 33/487*** $^{(2006.01)}$ ***B81B 1/00*** $^{(2006.01)}$

(86) International application number:
**PCT/IT2010/000382**

(87) International publication number:
**WO 2011/027379 (10.03.2011 Gazette 2011/10)**

(54) **NANOPORED SILICON NITRIDE CHIP FOR THE ANALYSIS OF GENE EXPRESSION PROFILES**

NANOPOREN-SILZIUMNITRIDCHIP ZUR ANALYSE VON GENEXPRESSIONSPROFILEN

PUCE DE NITRURE DE SILICIUM NANOPOREUSE POUR L'ANALYSE DE PROFILS D'EXPRESSION GÉNIQUE

(84) Designated Contracting States:
**AL AT BE BG CH CY CZ DE DK EE ES FI FR GB GR HR HU IE IS IT LI LT LU LV MC MK MT NL NO PL PT RO SE SI SK SM TR**

(30) Priority: **04.09.2009 IT RM20090450**

(43) Date of publication of application:
**11.07.2012 Bulletin 2012/28**

(73) Proprietor: **UNIVERSITA' DEGLI STUDI DI GENOVA**
**16126 Genova (IT)**

(72) Inventors:
- **TONINI, Gianpaolo**
  **I-16132 Genova (IT)**
- **SCARUFFI, Paola**
  **I-16132 Genova (IT)**
- **STIGLIANI, Sara**
  **I-16132 Genova (IT)**
- **VALBUSA, Ugo**
  **I-16126 Genova (IT)**
- **REPETTO, Luca**
  **I-16126 Genova (IT)**
- **FIRPO, Giuseppe**
  **I-16126 Genova (IT)**
- **MUSSI, Valentina**
  **I-16126 Genova (IT)**
- **FANZIO, Paola**
  **I-16126 Genova (IT)**

(74) Representative: **Gitto, Serena et al**
**Barzanò & Zanardo Roma S.p.A.**
**Via Piemonte, 26**
**00187 Roma (IT)**

(56) References cited:
- **WANUNU M ET AL: "Chemically modified solid-state nanopores", NANO LETTERS AMERICAN CHEMICAL SOCIETY USA, vol. 7, no. 6, June 2007 (2007-06), pages 1580-1585, XP7911217, ISSN: 1530-6984 cited in the application**
- **MUSSI V ET AL: "Solid state nanopores for gene expression profiling", SUPERLATTICES AND MICROSTRUCTURES, ACADEMIC PRESS, LONDON, GB, vol. 46, no. 1-2, 10 October 2008 (2008-10-10), pages 59-63, XP026152421, ISSN: 0749-6036 [retrieved on 2008-10-10] cited in the application**
- **HAN A ET AL: "Label-free detection of single protein molecules and protein-protein interactions using synthetic nanopores", ANALYTICAL CHEMISTRY 20080615 AMERICAN CHEMICAL SOCIETY US, vol. 80, no. 12, 15 June 2008 (2008-06-15) , pages 4651-4658, XP7911227,**
- **SMEETS R M M ET AL: "Translocation of RecA-coated double-stranded DNA through solid-state nanopores.", NANO LETTERS SEP 2009, vol. 9, no. 9, 3 December 2008 (2008-12-03), pages 3089-3096, XP7911221, ISSN: 1530-6992**
- **SMEETS R M M ET AL: "Noise in solid-state nanopores.", PROCEEDINGS OF THE NATIONAL ACADEMY OF SCIENCES OF THE UNITED STATES OF AMERICA 15 JAN 2008, vol. 105, no. 2, 15 January 2008 (2008-01-15), pages 417-421, XP7911228, ISSN: 1091-6490**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 473 849 B1

• IQBAL S M ET AL: "Solid-state nanopore channels with DNA selectivity", NATURE NANOTECHNOLOGY NATURE PUBLISHING GROUP UK, vol. 2, no. 4, April 2007 (2007-04), pages 243-248, XP7911218, ISSN: 1748-3387

**SEQUENCE LISTING**

<110> Istituto Nazionale per la Ricerca sul Cancro Università degli Studi di Genova

<120> Nanopored silicon nitride chip for the analysis of gene expression profiles and related biosensors

<130> PCT27960

<150> RM2009A000450
<151> 2009-08-04

<160> 2

<170> PatentIn version 3.5

<210> 1
<211> 1310
<212> DNA
<213> artificial

<220>
<223> gene housekeeping GAPDH

<400> 1

aaattgagcc cgcagcctcc cgcttcgctc tctgctcctc ctgttcgaca gtcagccgca 60
tcttcttttg cgtcgccagc cgagccacat cgctcagaca ccatggggaa ggtgaaggtc 120
ggagtcaacg gatttggtcg tattgggcgc ctggtcacca gggctgcttt taactctggt 180
aaagtggata ttgttgccat caatgacccc ttcattgacc tcaactacat ggtttacatg 240
ttccaatatg attccaccca tggcaaattc catggcaccg tcaaggctga gaacgggaag 300
cttgtcatca atggaaatcc catcaccatc ttccaggagc gagatccctc caaaatcaag 360
tggggcgatg ctggcgctga gtacgtcgtg gagtccactg gcgtcttcac caccatggag 420
aaggctgggg ctcatttgca ggggggagcc aaaagggtca tcatctctgc cccctctgct 480
gatgccccca tgttcgtcat gggtgtgaac catgagaagt atgacaacag cctcaagatc 540
atcagcaatg cctcctgcac caccaactgc ttagcacccc tggccaaggt catccatgac 600
aactttggta tcgtggaagg actcatgacc acagtccatg ccatcactgc cacccagaag 660
actgtggatg gcccctccgg gaaactgtgg cgtgatggcc gcggggctct ccagaacatc 720
atccctgcct ctactggcgc tgccaaggct gtgggcaagg tcatccctga gctgaacggg 780
aagctcactg gcatggcctt ccgtgtcccc actgccaacg tgtcagtggt ggacctgacc 840
tgccgtctag aaaaacctgc caaatatgat gacatcaaga aggtggtgaa gcaggcgtcg 900
gagggccccc tcaagggcat cctgggctac actgagcacc aggtggtctc ctctgacttc 960
aacagcgaca cccactcctc cacctttgac gctggggctg gcattgccct caacgaccac 1020
tttgtcaagc tcatttcctg gtatgacaac gaatttggct acagcaacag ggtggtggac 1080
ctcatggccc acatggcctc caaggagtaa gacccctgga ccaccagccc cagcaagagc 1140
acaagaggaa gagagagacc ctcactgctg gggagtccct gccacactca gtcccccacc 1200
acactgaatc tcccctcctc acagttgcca tgtagacccc ttgaagaggg gaggggccta 1260

gggagccgca ccttgtcatg taccatcaat aaagtaccct gtgctcaacc 1310

<210> 2
<211> 45
<212> DNA
<213> artificial

<220>
<223> gene housekeeping GAPDH functionalization probe

<400> 2
gccaaaaggg tcatcatctc tgcccctct gctgatgccc ccatg 45

## Description

The present invention concerns a biosensor with a nanopored silicon nitride chip for the analysis of gene expression profiles and related biosensors. More particularly, the present invention concerns a biosensor to be used for diagnosis and prophylaxis in medical, botanical, zoo-technical and zoo-prophylactic field.

The Human Genome project allows new genes and expressed sequences to be identified, this project will allow the human genome complete de-codification (http://www.ornl.gov/hgmis/). At the same time new technologies for genome analysis, in particular the microarray technology, allowing tens of thousands of genes in a single experiment to be analysed, have been developed. Microarray technology is based on the following principle: single strand DNA molecules are fixed to a solid support, such probes are suitable to hybridize to complementary DNA sequences. Such microarray devices are used for the characterization of gene expression profiles, in order to identify point mutations and polymorphisms of single nucleotides, in order deleted or inserted chromosome regions to be identified [1].

Recently [2], for the genome analysis the nanopore technology has been developed. The use of nanopores for the detection and analysis of single molecules, is derived from Coulter counter principle [3]: a small channel separating two reservoirs filled with an electrolytic solution wherein target particles are dissolved. By application a voltage difference within the channel, the particles are forced to cross the same and passage thereof is evidenced by a current drop. Current decrease magnitude is directly correlated to particle volume. This analytical technique therefore allows the concentration and size distribution of dispersed particles to be determined.

The results reported in [2] demonstrate the possibility to scale down this behaviour to nanometric size using pores of $\alpha$-hemolysin, a protein produced by *Staphilococcus aureous,* able to self-assembling within double lipid layer of biological membranes, forming a pore with a diameter of 1.4 nm.

A successive step in this direction has been made according to [4] by identification of DNA strands with a single base resolution using "engineered nanopores". This object has been reached by covalenty binding DNA-oligonucleotides (probes) near the entrance of pore lumen, in this way the pore is partially obstructed and ionic conductivity thereof is reduced. When a short complementary oligonucleotide (target) is forced through the pore lumen by the applied voltage, said oligonucleotide forms a double strand DNA with the probe, resulting in a characteristic current reduction. A not complementary oligonucleotide is pushed quickly through pore under the voltage application, producing a marked reduction of the current thus evidencing the translocation without double strand formation. In this way the device with biological engineered nanopore is used to evidence a codon in a single DNA molecule [4], or to evidence a difference between molecules at single nucleotide level [5].

Although it has been proved that biological pores are useful in a large number of applications, the same also show remarkable disadvantages correlated mainly to fixed size and limited stability thereof. For this reason various research teams are involved in the possible production of nanopores from solid state materials, resulting in many advantages, as: 1) high stability, 2) control of channel diameter and length, 3) adjustable surface properties and 4) possibility for nanopores to be integrated in devices and arrays [6,7]. As to the production of solid state nanopores as described for example in [6] and [7] the same show, in turn, some disadvantages, as for example the need controlled ionic sputtering process (ref [6]) or electron beam scanning (ref [7]) to be used in order to reduce the final size of the pore initially created using ionic focused beams.

In the light of above it is therefore apparent the need to provide for new devices for the detection of nucleotide sequences thus overcoming the disadvantages of known prior art.

The authors of the present invention previously have prepared solid state nanopores, functionalized, activated with known sequence DNA molecules, obtained using ionic beams within a suspended thin silicon nitride membrane ($Si_3N_4$) [8]. It has been therefore demonstrated that solid state nanopores can be stably functionalized with DNA molecules. However, the parameters of previously prepared biosensors have not been displayed effective for the detection of oligonucleotide molecule passage, even proved to be quite incompatible with the measure of translocation thereof.

The Authors of the present invention have now provided for solid state nanopores allowing the stable use thereof as selective biosensors suitable to recognize specific nucleotide sequences within unknown DNA molecule to be carried out. According to the present invention it is therefore possible solid state nanopores to be functionalized effectively with DNA, thus to be made the same more stable and less sensitive to environmental conditions than biological ones. The term "to functionalize effectively" means not so much to find chemical procedures aiming DNA to be bound to $Si_3N_4$, which is original but not completely new, as to be suitable to calibrate in a correct way the characteristic parameters of the problem, as the relationship of the length of DNA chosen molecules and the nanopore geometric dimensions, the concentrations of DNA molecules and ionic solution used for the measures of current through the nanopore and so on.

In comparison to other procedures for DNA adhesion to nanopore surface, herein disclosed one does not involve the local pre-deposition on nanometric areas of material (gold, platinum, oxide etc), said pre-deposition requiring complex and expensive processes of lithographic type thus increasing times, costs and failure risks during the chip preparation, to be carried out.

Secondly nanopore initial size (higher than 30 nm) allows FIB alternative lithographic production techniques to be provided, that is at remarkably reduced costs.

Moreover the same procedure for chemical functionalization can be used independently from the sequence of oligonucleotide probe. Therefore, a single chip can be used as an array of nanopores, each thereof being differently functionalized in order a specific sequence to be recognised. A device of this type represents a very powerful diagnostic tool. Said device in fact, also being similar, as to "hybridization" operation principle, to a micro-array, involves an extremely quick and simple reading, that is a current measure rather than an optical type measure.

In comparison to same type biosensor consisting of biological nanopore, the inventive one displays advantages with respect to stability, modifiability, suitability to be integrated in more complex devices, re-usability. Moreover, the biosensor according to the invention can operate at extreme temperatures and pH ranges and are easily stored.

The silicon nitride based nanopore device functionalized with oligonucleotide probes according to the invention can be advantageously used for the analysis of expression profiles for research, diagnostic and prognostic purposes. The device according to the invention allows, in various pathologies (cancerous and not), the profiles of gene expression from various origin biological (bioptic material, peripheral or medullary blood etc) samples to be analysed. Such application allows molecular markers with prognostic meaning to be evaluated and/or a better patient staging within the risk categories and the consequent therapy personalization to be carried out. Particularly, the device allows SNP to be identified in biological samples relating to various pathologies (cancerous and not). Being known that SNPs are associated to susceptibility of some diseases and different response to pharmacological therapies, such an analysis will allow a patient fingerprinting to be carried out aiming the pathology predisposition and drug response to be estimated. Moreover, the device according to the invention can be used for applications in botanical, zoo-technical and zoo-prophylactic fields, for example for the identification of OGM in foods or growing crops or virus characterization.

The present invention provides a biosensor comprising a chip comprising a nanopored silicon nitride membrane, wherein nanopores have an effective diameter from 20 to 50 nm and the thickness of the membrane is from 10 nm to 50 nm and more preferably from 10 to 20 nm, preferably produced by means of focused ion beams, for example by means of the use of ultra-high resolution field emission scanning electron microscope with focused ion beam.

At least one nanopore is functionalized with an oligonucleotide having a length from 13.6 nm to 18.7 nm, preferably 15.3 nm. The nanopores can be functionalized with the same or different oligonucleotides. The effective diameter of functionalized nanopores of the biosensor varies from 3 to 20 nm, preferably from 4 to 10 nm, still more preferably from 4 to 6 nm.

It is a further object of the invention a process for the preparation of as above described biosensor comprising or consisting of the following steps: a) deposition of a solution of oligonucleotide having a length from 40 to 55 bases, preferably 45 bases, said solution having a concentration from 10 to 200 nM, preferably 100 nM, in bi-distilled water or SSC 2X that is a sodium chloride 0.3 M solution, 0.03 M sodium citrate at pH 7.1; b) deactivation of the biosensor surface. The deactivation of the biosensor surface can be carried out by means of washings with 28% ammonia solution and successive washings with bi-distilled water.

The present invention concerns moreover a device for the detection of oligonucleotide sequences comprising the biosensor as above defined. The device can further comprise two ionic solution containing reservoirs communicating through the nanopores and two electrodes for voltage application and current detection.

The use of the biosensor or device as above defined for oligonucleotide molecule detection in medical, botanical, zoo-technical or zoo-prophylactic fields is a further object of the present invention.

The present invention now will be described by an illustrative, but not limitative way, according to preferred embodiments thereof, with particular reference to enclosed drawings wherein:

Figure 1 shows a cell scheme according to A J Storm, J H Chen, H W Zandbergen, and C Dekker, Translocation of double-strand dna through a silicon oxide nanopore. Physical Review, 71:051903, 2005 [9].

Figure 2 shows the cell of figure 1 located on antivibrating table inside of a Faraday double cage.

Figure 3 shows a current ramp detected by applying different voltages to the measuring cell containing nanopored chip (figure 3A) and the voltage-current curve extrapolated from current ramp with dotted line outlined linear fit in order R nanopore resistance to be estimated (the figure 3B).

Figure 4 shows a nanopore scheme wherein r is the radius and 1 the length.

Figure 5 shows on the left (A) a schematic representation of experimental conditions. In the lower part the nanopored chip with attached oligonucleotide probe is represented. Moreover DNA target molecules which tend to the translocation into the pore under the action of applied voltage are represented. On the right (b) a schematic representation of ionic current signal which is likely to be expected is reported.

Figure 6 shows a picture according to S.Howorka, S. Cheley, and H.Bayley, Sequence-specific detection of individual DNA strands using engineered nanopores, Nature Biotechnology, 19 (2001) 636-639 [4]. On the left the configuration of perfect complementariness between nanopore attached DNA probe (in gray) and trans-locating target DNA (in black); on the right translocation in the presence of two filament mismatch. In the lower part, in the picture, respective

measured ionic current signals are represented.
Figure 7 shows a scheme according to S. M. Iqbal, D. Akin, and R.Bashir, Nature Nanotechnology, 2:243-248, 2007 [10]. The oxide layer to be deposited on nanochannel Si in order the surface functionalization with "hairpin" DNA molecules to be carried out. Moreover passing target DNA is shown.
Figure 8 shows a scheme according to M. Wanunu and A. Meller, Chemically modified solid-state nanopores, Nano Letters, 7(6):1580-1585, 2007 [11]. Functionalization organic molecules are represented.
Figure 9 shows a schematic representation of starting $Si/Si_3N_4$ chip.
Figure 10 shows the current ramp obtained on a nanopored chip in range 0-55 mV.
Figure 11 shows the linear Fit of the measure reported in figure 8, from which it results R = 6.91 MΩ.
Figure 12 shows GAPDH housekeeping Gene (SEQ ID NO:2). In gray colour sequence (SEQ ID NO: 1) of functionalization molecule probe is reported.
Figure 13 shows the linear Fit of current measures before (black squares) and after (gray circles) the functionalization with oligonucleotide probe.
Figure 14 shows pictures obtained using a scanning electron microscope (SEM) of a nanopored chip before (on the left) and after (on the right) the functionalization with oligonucleotide probe.
Figure 15 shows the scheme of oligonucleotide molecule not functionalized (A) and functionalized nanopore.
Figure 16 shows the microfluid cell for the measures of nanopore current.
Figure 17 shows the chip section dipped within ionic solution. Oligonucleotide probes are fixed to the chip. In the cis reservoir, λ-DNA target molecules directed to pore under the application of voltage V are dipped.
Figure 18 shows the measured ionic current during the experiment of molecular translocation described in the "material and methods" section. B and C: Successive magnifications of A plot shown in order to appreciate single current spikes associated to the passage through nanopore of λ-DNA molecules. In figure C average translocation duration is indicated, equal to approximately 1.36 ms when 120 mV voltage is applied and cis and trans reservoirs are filled with KCl 2 M and 0.01 M, respectively.

EXAMPLE 1: *Preparation and operation of the biosensor according to the invention*

According to preferred embodiments, the fundamental components of the measure apparatus are a cell consisting of two reservoirs containing ionic solution (typically KCl 1 M), in communication only through the nanopore, and two electrodes (chlorinated silver wire Ag/AgCl) for the voltage application and current detection (figure 1).

The cell, made of polymer (PDMS) or plexiglass, is located on an antivibrating table inside of a double Faraday cage so as to reduce the electrical and mechanical noise (figure 2).

The measures of ionic current therefore are carried out inserting the nanopored chip in the measure cell and applying a voltage difference between the two solution dipped electrodes. Particularly voltage ramps are applied and current ramps are recorded (figure 3A) in order voltage-current curves to be plotted. From the latter, with a linear fit to delineate (figure 3B) it is possible to have an evaluation of nanopore R resistance.

By using system exemplification hypotheses, from nanopore R resistance R an evaluation of diameter thereof can be obtained. If we assume that nanopore is substantially a cylindrical channel with length 1 equal to the thickness of the membrane whereon the pore is made it is obtained (Figure 4):

$$R= 1/\sigma\pi r^2$$

where σ is the conductivity of the ionic solution and R is the radius of cylindrical pore that therefore can be evaluated directly by the measure of R.

Nanopored chip can be functionalized for the realization of a biosensor suitable to experiments for gene expression analysis. The nanopore is functionalized by fixing known sequence "probe" oligonucleotides, to surface thereof, thus it can be used as biosensor in applications of gene expression profiling, SNP search or mutations. Target unknown DNA molecules are inserted in the reservoir wherein the negative electrode is dipped and, due to negative charge thereof, will tend to cross the nanopore, after the application of a voltage difference between two sides of the nanopored chip (figure 5A). During the nanopore crossing the target molecules cause a partial clogging of the pore, that is a reduction of the level of measured ionic current. If probe and target filaments are complementary during the crossing the molecules will be slowed, staying longer in the channel and therefore causing a longer lasting clogging (figure 5B).

Analogous measures have been already carried out with biological nanopores (see figure 6), and various techniques have been already developed in order $SiO_2$ and $Si_3N_4$ made solid state nanopores to be functionalized with "DNA hairpin" (figure 7) and aminosilanes, respectively (figure 8).

Chips used for the invention consist of a silicon substrate whereon, in succession, thin films of silicon oxide

and silicon nitride, the latter being 20 nm thick, are coated. Using a chemical etching process the chip is excavated from the bottom in order the suspended silicon nitride membrane to be exposed resulting in an approximately 80 x 80 μm wide window. On the upper part of the chip it is possible, but not necessary, to carry out metal coatings to be used as local electrodes.

The chips can be nanopored using an Ultra High Resolution Field Emission Scanning Electron Microscope (UHR-FE-SEM) with Focused Ion Beam (FIB) allowing, using ionic sputtering, nanopores of diameter from 30 nm to 50 nm on a suspended thin silicon nitride membrane ($Si_3N_4$) to be produced. For the measure of ionic current the nanopore is inserted in a microfluid cell made of plexiglass and inserted in a double Faraday cage on an antivibrating table. The reservoirs of the measurement chamber are filled with a KCl 1M HEPES 10 mM buffered solution at pH 8. The measure is carried out using two Ag/AgCl electrodes used both for the voltage application and current detection by means of patch-clamp amplifier (Axopatch 200B, Axon Instruments).

Figure 10 reports the standard measure carried out on a nanopored chip. From the measure it is possible to obtain, as above said, an evaluation of nanopore R electric resistance by a linear fit (figure 11).

From the R value it is deduced, based on (1), an evaluation of $d_{eff}$ nanopore effective diameter, approximated to a cylindrical channel with length 1 (1 = 20 nm) equal to the thickness of silicon nitride membrane and conductivity σ equal to that of KCl 1M solution (10 S/m):

$$d_{eff}=2\cdot\sqrt{\frac{l}{\sigma\cdot\pi\cdot R}}$$

R = 2.52 MΩ --→ d = about 32 nm circa

At this point the chemical functionalization of nanopored chips with known sequence oligonucleotide can involve the application of the following protocol:

1. Substrate pre-treatment:

- Substrate washing with $ddH_2O$;
- Treatment of the substrate with amino propyl triethoxy-silane (20% solution of $ddH_2O$) 1 hour at room temp.;

2. Substrate activation:

- Treatment of the substrate with 1,4-phenylene-diisothiocyanate (5% solution of dimethylsulfoxide) 5 hours at room temp.;
- Substrate washing with dimethylsulfoxide (2 times);
- Substrate washing with $ddH_2O$ (2 times);
- Air substrate drying at room temp.;

3. DNA adhesion:

- Manual deposition of 100 nM oligonucleotide (45mer) solution of ddH2O;
- Incubation at 37°C O/N in humid box.

4. Substrate deactivation

- Surface washing with 28% ammonia (2 times 30 minutes each) Surface washing with ddH2O (2 times 15 minutes each)

5. Chip storage

- Vacuum and refrigerator stored at 5°C.

The above described protocol can be diagrammatically represented as below:

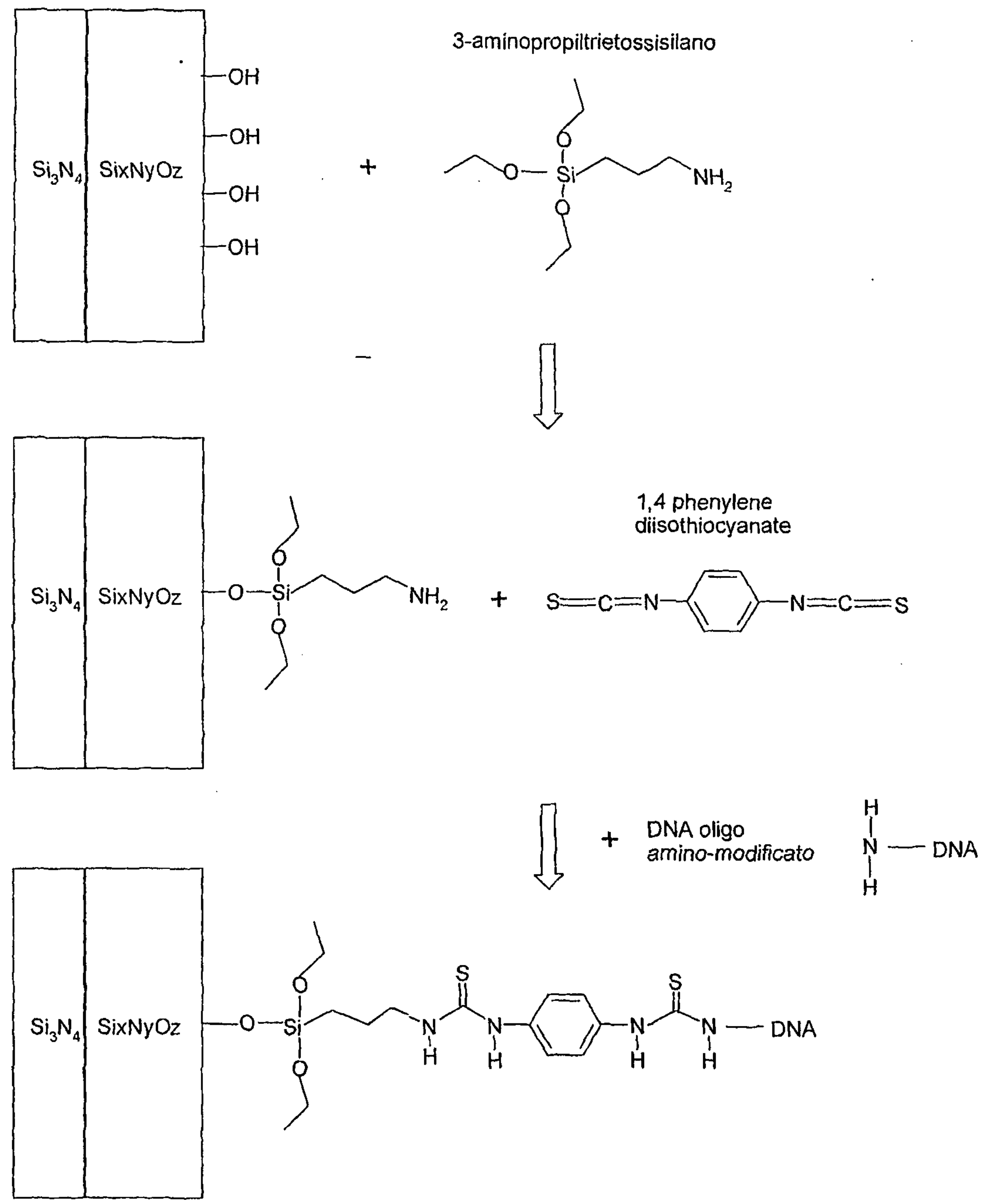


3-amminopropiltrietossisilano= 3-amino propyltriethoxysilane, amino-modified oligo DNA

By the application of functionalization protocol therefore a nanopore with known sequence probe DNA molecules fixed to the active surface can be obtained. Particularly we used an oligo DNA extracted (SEQ ID NO: 1) from GAPDH "housekeeping" gene (Homo sapiens glyceraldehyde-3-phosphate dehydrogenase) (SEQ ID NO: 2), expressed by all the cells. It is the sequence of 45 bases (15.3 nm long filaments) gray coloured in figure 12.

The successful functionalization can be checked using ellipsometric fluorescence based techniques or verifying that the functionalization results in nanopore size narrowing, that is a reduction of the effective diameter resulting in an increase of electric resistance. Figure 13 shows in gray colour (circles) the current value obtained for nanopore as compared with that (black squares) obtained before the functionalization and also reported in figure 11.

Therefore the functionalization with 15.3 nm long probe molecules results in a reduction of pore diameter of approximately 25.5 nm (32 - 6.5 nm), that is approximately 13 nm of radius obstruction (probe molecules do not assume a completely flat configuration) .

The presence of probe molecules after the functionalization has been also verified by SEM images (figure 14). Also from SEM images it is possible to evaluate a lateral obstruction by functionalization organic layer of approximately

11 nm. It is an obstruction that results in a reduction of nanopore effective diameter from 30-40 nm to 8-18 nm, that is an average value allowing the final hybridization tests of probe molecules with pore translocating target DNA molecules to be effectively carried out (where "effectively" means the occurrence of a detectable reduction of ionic current average level during the translocation). The current measure calibrated functionalization protocol represents the first step (the most important) for the production of a biosensor for target sequences.

In summary the patent idea is correlated with the possibility of taking advantage of the functionalization its-self in order to not only make the nanopore selective for the specific analyzed probe-target interaction, but also in order to reduce the size of the same pore up to a value compatible with the obstruction of individual target molecules in solution.

This means to be in a contest as schematized in figure 15A, that is as a pore with much greater size than analyzed DNA molecules and therefore insensitive to the passage of bio-molecules and obtain, by means of functionalization, the result as reported in 15B, i.e. a pore with a reduced final effective diameter up to a value compatible with individual molecule analysis. A thus realized device allows "to see" passing DNA molecules as current reduction transient events.

The signal to noise ratio of the device, that is the relationship between the amplitude of current modulation due to the passage of molecules, $\Delta I$ ("Event amplitude" in figure 1B), and average ionic current I, depends on the ratio of target molecule section, $\Delta A$, to pore effective section, A, according the relation:

$$\frac{\Delta I}{I} = \frac{A_{DNA}}{A}$$

The latter, based on the Ohm law (V = R I), can be written, as:

$$\Delta I = I\frac{A_{DNA}}{A} = \frac{V}{R}\frac{A_{DNA}}{A} = \frac{V\sigma A}{L}\frac{A_{DNA}}{A} = \frac{V\sigma A_{DNA}}{L}$$

where $\sigma$ is the conductivity of used ionic solution, V is the applied voltage and L the membrane thickness (that is the length of nanochannel).

Therefore the occurrence of molecule passage is detectable at sufficiently high voltage and conductivities and sufficiently low membrane thickness. Particularly, given the intrinsic noise level of the device based on silicon nitride nanopores (amplitude of the order of 300 pA), our results show that, in normal conditions, the passage of molecules is detectable as a recognizable event from noise background only for membrane thickness lower than 50 nm. As a demonstration in example 2 the result of a check experiment for single molecule sensitivity of functionalized nanopore is reported.

<u>EXAMPLE 2:</u> *Experimental examination of single molecule sensibility of nanopore device functionalized according* to the invention

Materials and methods

$Si/Si_3N_4$ chip as represented in figure 9 consisting of silicon support 300 $\mu$m thick and coated with ~ 10 nm thick silicon nitride ($Si_3N_4$) film. The silicon support has been subjected to anisotropic etching in order 80x80 $\mu m^2$ $Si_3N_4$ wide area to be exposed. On this area, constituting a suspended membrane, a single nanopore with nm effective diameter has been produced. Then the nanopore has been subjected to functionalization procedure as described in example 1 protocol, using 45-mer oligonucleotides. Particularly 5'-amino modified 45-mer oligonucleotides (MWG Biotech, Germany) corresponding to 5'-GCC AAA AGG GTC ATC ATC TCT GCC CCC TCT GCT GAT GCC CCC ATG-3' (SEQ ID NO:1) sequence of *GAPDH* (glyceraldehyde-3-phosphate dehydrogenase) gene have been used.

Chip containing the functionalized nanopore has been assembled in a microfluid cell for ionic current measurements. Figure 16 represent the plexiglass made cell and consisting of two ionic solution reservoirs faced at chip chamber and equipped with channels for inlet and outlet of ionic solution and pores for Ag/AgCl electrode positioning. The chip chamber is equipped with two rubber seals.

The silver/silver chloride electrodes are connected to a patch-clamp amplifier (Axopatch 200B, Axon Instruments) for voltage (120 mV) application and nanopore current detection. The current detection is carried out with 200khz sampling rate and using a 2$^{nd}$ order Bessel filter at 10 khz. For the measurements a potassium chloride solution, KCl,

at 2 M concentration in cis reservoir, i.e. wherein initially target molecules have been dispersed and at 0.01 M concentration in trans reservoir (see figure 17).

Experiment target molecules are double strand ~ 48 kbp λ-DNA molecules.

Results

Given the background noise level of device based on silicon nitride nanopores (amplitude of the order of 300 pA), our results show that, under normal conditions, the molecule passage is detectable as background noise recognizable event only for membrane thicknesses lower than 50 nm. As a demonstration in figure 18 results of molecular translocation experiment carried out under conditions illustrated in above reported material and methods section are reported: on the current plot spikes associated to the passage of single DNA target molecules not found using nanopores not functionalized or functionalized by means of membranes having thickness equal or lower than 50 nm, appear. In figure (18C) are also reported the average translocation durations, equal to approximately 1.36 ms. This value is directly related to the length of target molecule and interaction level of target with probe molecules.

References

[1] M. Schena, Microarray biochip technology. Eaton Publishing, 2000.
[2] J. J. Kasianowicz, E. Brandin, D. Branton, D.W. Deamer, Procl. Natl. Acad. Sci. USA 93 (1996) 13770-13773.
[3] WH. Coulter, Proceedings of the National Electronics Conference (1957) 1034-1042.
[4] S. Howorka, S. Cheley, H. Bayley, Nature Biotechnology 19 (2001) 636-639.
[5] S. Winters-Hilt, M. Landry, M. Akeson, M. Tanase, I. Amin, A. Coombs, E. Morales, J. Millet, C. Baribault, S. Sendamangalam, Cheminformatics BMC Bioinformatics 7 (2006) S22.
[6] J. Li, D. Stein, C. McMullan, D. Branton, M. J. Aziz, J.A. Golovchenko. Nature 412 (2001) 166-169.
[7] C. J. Lo, T. Aref, A. Bezryadin, Nanotechnology 17 (2006) 3264-3
[8] Mussi et al., Superlattices and Microstructures, vol. 46, no. 1-2, 10 October 2008, pages 59-63.
[9] A J Storm, J H Chen, H W Zandbergen, and C Dekker. Physical Review E, 71:051903, 2005.
[10] S. M. Iqbal, D. Akin, and R.Bashir, Nature Nanotechnology, 2:243-248, 2007
[11] M. Wanunu and A. Meller, Nano Letters, 7(6):1580-1585, 2007.

## Claims

1. Biosensor for the analysis of gene expression profiles comprising a chip, said chip comprising a nanopored silicon nitride membrane having nanopores with effective diameter from 20 to 50 nm, at least one nanopore being functionalized with an oligonucleotide complementary to a target sequence, said oligonucleotide having a length from 13.6 nm to 18.7 nm, preferably 15.3 nm, the effective diameter of the functionalized nanopores being from 3 to 20 nm, preferably from 4 to 10 nm, still more preferably from 4 to 6 nm;

said effective diameter being defined as

$$d_{eff}=2\cdot\sqrt{\frac{l}{\sigma\cdot\pi\cdot R}}$$

wherein R is the nanopore electric resistance, 1 is the membrane thickness and $\sigma$ is the conductivity of a ionic solution in which the nanopore is dipped,
**characterised in that** the silicon nitride membrane has a thickness from 10 nm to 50 nm, preferably from 10 nm to 20 nm.

**2.** Biosensor according to claim 1, wherein the nanopores are functionalized with the same or different oligonucleotides.

**3.** Process for the preparation of the biosensor according to anyone of claims 1-2 comprising the following steps:

a) treatment of the nanopored silicon nitride membrane with a 20% solution of amino propyl-triethoxy-silane in dd$H_2$O for 1 hour at room temperature and then with a 5% solution of 1,4-phenylene20 diisothiocyanate in dimethylsulfoxide for 5 hours at room temperature;
b) deposition of a solution of oligonucleotide having a length from 40 to 55 bases, preferably 45 bases, said solution having a concentration from 10 to 200 nM, preferably 100 nM, in bi-distilled water or sodium chloride 0.3 M solution, 0.03 M sodium citrate and pH 7.1; c) deactivation of the biosensor surface.

**4.** Process according to claim 3, wherein the deactivation of the biosensor surface occurs by means of washings with a 28% ammonia solution and successive washings with bi-distilled water.

**5.** Device for detection of DNA molecule comprising the biosensor as defined in anyone of the claims 1-2.

**6.** Device according to claim 5, further comprising two ionic solution containing reservoirs communicating through the nanopores and two electrodes for voltage application and current detection.

**7.** Use of a biosensor as defined in anyone of claims 1-2 or a device as defined in each of claims 5-6 for DNA molecule detection in medical, botanical, zoo-technical or zoo-prophylactic fields.

## Patentansprüche

**1.** Biosensor zur Analyse von Genexpressionsprofilen, welcher einen Chip umfasst, wobei der Chip eine nanoporige Siliciumnitrid-Membran mit Nanoporen eines effektiven Durchmessers von 20 bis 50 nm umfasst, wobei mindestens eine Nanopore mit einem Oligonukleotid funktionalisiert ist, welches komplementär zu einer Targetsequenz ist, wobei das Oligonukleotid eine Länge von 13,6 nm bis 18,7 nm, vorzugsweise 15,3 nm aufweist, der effektive Durchmesser der funktionalisierten Nanoporen 3 bis 20 nm, vorzugsweise 4 bis 10 nm, bevorzugter 4 bis 6 nm beträgt; wobei der effektive Durchmesser definiert ist als

$$d_{eff}=2\cdot\sqrt{\frac{l}{\sigma\cdot\pi\cdot R}}$$

wobei R der elektrische Widerstand der Nanopore ist, 1 die Dicke der Membran ist und $\sigma$ die Leitfähigkeit einer ionischen Lösung ist, in welche die Nanopore eingetaucht ist, **dadurch gekennzeichnet, dass** die Siliciumnitrid-Membran eine Dicke von 10 nm bis 50 nm, vorzugsweise 10 nm bis 20 nm, aufweist.

**2.** Biosensor nach Anspruch 1, wobei die Nanoporen mit den gleichen oder unterschiedlichen Oligonukleotiden funktionalisiert sind.

**3.** Verfahren zur Herstellung des Biosensors nach irgendeinem der Ansprüche 1-2, umfassend die folgenden Schritte:

a) Behandlung der nanoporigen Siliciumnitrid-Membran mit einer 20%igen Lösung von Aminopropyltriethoxysilan in bidestilliertem $H_2O$ für eine Stunde bei Raumtemperatur und dann mit einer 5%igen Lösung von 1,4-Phenylendiisothiocyanat in Dimethylsulfoxid für 5 Stunden bei Raumtemperatur;
b) Abscheidung einer Lösung von Oligonukleotid mit einer Länge von 40 bis 55 Basen, vorzugsweise 45 Basen, wobei die Lösung eine Konzentration von 10 bis 200 nM, vorzugsweise 100 nM, in bidestilliertem Wasser oder einer Lösung von 0,3 M Natriumchlorid, 0,03 M Natriumcitrat bei einem pH-Wert von 7,1 aufweist;
c) Deaktivierung der Biosensor-Oberfläche.

**4.** Verfahren nach Anspruch 3, wobei die Deaktivierung der Biosensor-Oberfläche mittels Waschungen mit einer 28%igen Ammoniaklösung und sukzessiver Waschungen mit bidestilliertem Wasser geschieht.

**5.** Vorrichtung zum Nachweis eines DNA-Moleküls, umfassend den Biosensor wie in irgendeinem der Ansprüche 1-2 definiert.

**6.** Vorrichtung nach Anspruch 5, ferner umfassend zwei eine ionische Lösung enthaltende Reservoire, welche durch die Nanoporen kommunizieren, und zwei Elektroden zur Anlegung einer Spannung und zum Nachweis von Strom.

**7.** Verwendung eines Biosensors wie in irgendeinem der Ansprüche 1-2 definiert oder einer Vorrichtung wie in irgendeinem der Ansprüche 5-6 definiert zum Nachweis eines DNA-Moleküls auf dem Gebiet der Medizin, Botanik, Zootechnik oder Zooprophylaxe.

**Revendications**

**1.** Biocapteur pour l'analyse de profils d'expression de gènes comprenant une puce, ladite puce comprenant une membrane en nitrure de silicium à nanopores ayant des nanopores avec un diamètre efficace compris de 20 à 50 nm, au moins un nanopore étant fonctionnalisé avec un oligonucléotide complémentaire à une séquence cible, ledit oligonucléotide ayant une longueur comprise de 13,6 nm à 18,7 nm, et, de préférence, de 15,3 nm, le diamètre efficace des nanopores fonctionnalisés étant compris de 3 à 20 nm, de préférence de 4 à 10 nm, et de façon encore plus préférable de 4 à 6 nm ;
ledit diamètre efficace étant défini par :

$$d_{eff} = 2.\sqrt{\frac{l}{\sigma.\pi.R}}$$

où R est la résistance électrique du nanopore, l est l'épaisseur de la membrane et $\sigma$ est la conductivité d'une solution ionique dans laquelle le nanopore est trempé,
**caractérisé en ce que** la membrane en nitrure de silicium a une épaisseur comprise de 10 nm à 50 nm, et de préférence de 10 nm à 20 nm.

**2.** Biocapteur selon la revendication 1, dans lequel les nanopores sont fonctionnalisés avec le même oligonucléotide ou avec des oligonucléotides différents.

**3.** Procédé pour la préparation du biocapteur selon l'une quelconque des revendications 1 à 2, comprenant les étapes suivantes : a) le traitement de la membrane en nitrure de silicium à nanopores avec une solution à 20% d'amino propyl-triéthoxy-silane dans dd$H_2O$ pendant 1 heure à la température ambiante, puis avec une solution à 5% de 1,4-phénylène 20 diisothiocyanate dans du diméthylsulfoxyde pendant 5 heures à la température ambiante ; b) la déposition d'une solution d'oligonucléotide ayant une longueur de 40 à 55 bases, et, de préférence, de 45 bases, ladite solution ayant une concentration comprise de 10 à 200 nM, et, de préférence, de 100 nM, dans de l'eau bidistillée ou une solution de chlorure de sodium à 0,3 M, du citrate de sodium à 0,03 M, et un pH de 7,1 ; c) la désactivation de la surface du biocapteur.

**4.** Procédé selon la revendication 3, dans lequel la désactivation de la surface du biocapteur se produit à l'aide de lavages avec une solution d'ammoniac à 28 % et de lavages successifs avec de l'eau bidistillée.

**5.** Dispositif pour la détection d'une molécule d'ADN, comprenant le biocapteur selon l'une quelconque des revendications 1 à 2.

**6.** Dispositif selon la revendication 5, comprenant de plus deux réservoirs contenant une solution ionique communiquant par l'intermédiaire des nanopores et deux électrodes pour une application de tension et une détection de courant.

**7.** Utilisation d'un biocapteur selon l'une quelconque des revendications 1 à 2 ou dispositif selon chacune des revendications 5 à 6 pour une détection de molécule d'ADN dans les domaines médical, zootechnique ou zoo-prophylactique.

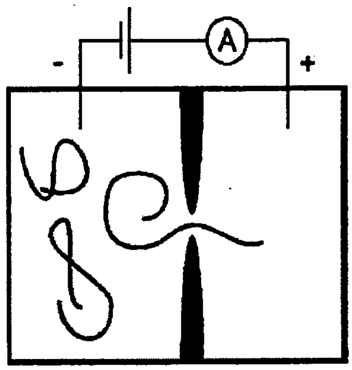
-
+
A

Fig. 1

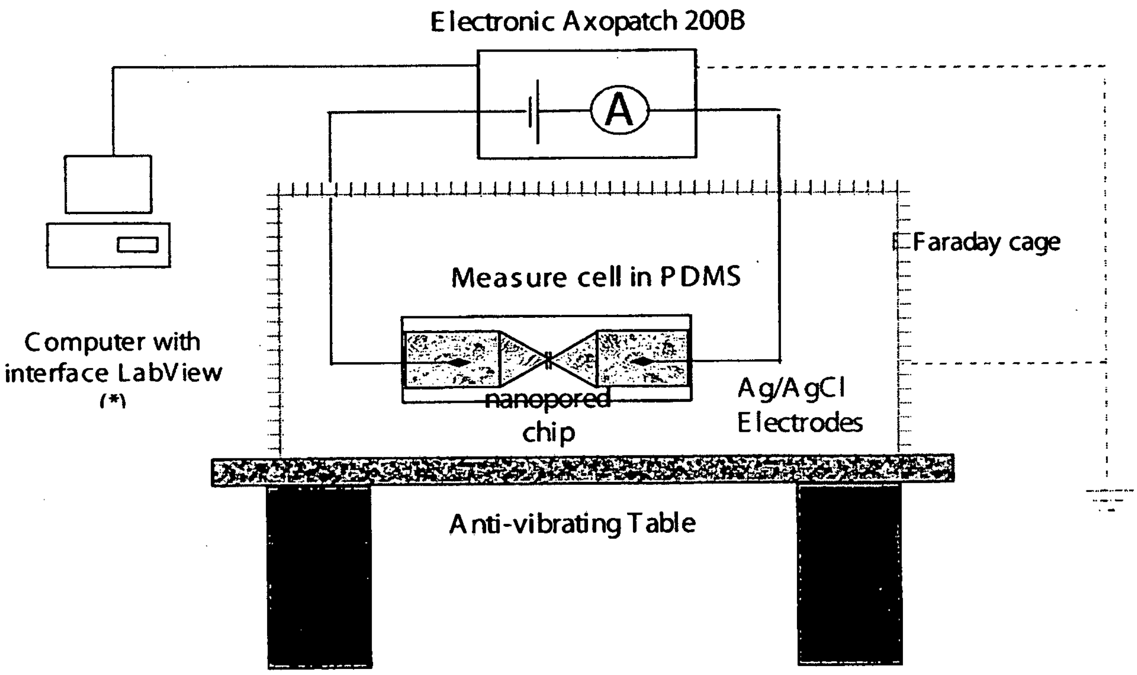
Electronic Axopatch 200B
A
Faraday cage
Measure cell in PDMS
Computer with
interface LabView
(*)
nanopored
chip
Ag/AgCl
Electrodes
Anti-vibrating Table

Fig. 2

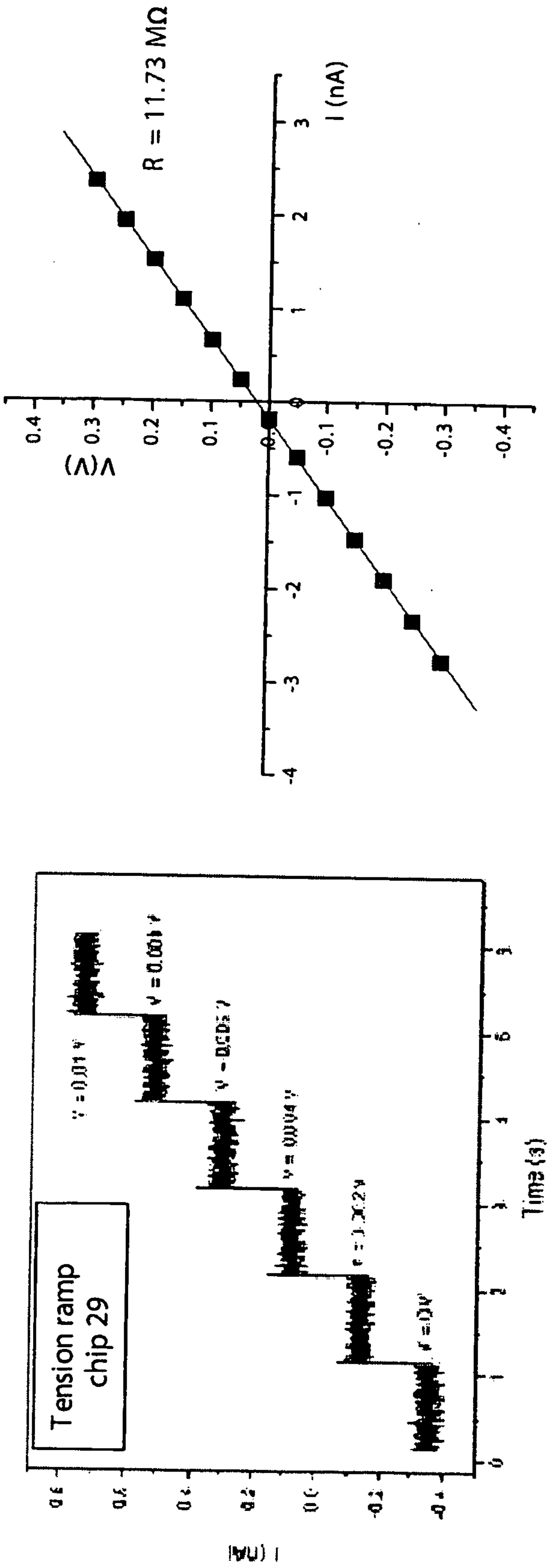


Fig. 3

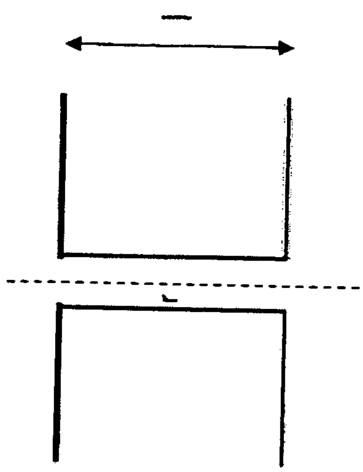


$$R = \frac{l}{\sigma \pi r^2} \quad (1)$$

Fig. 4

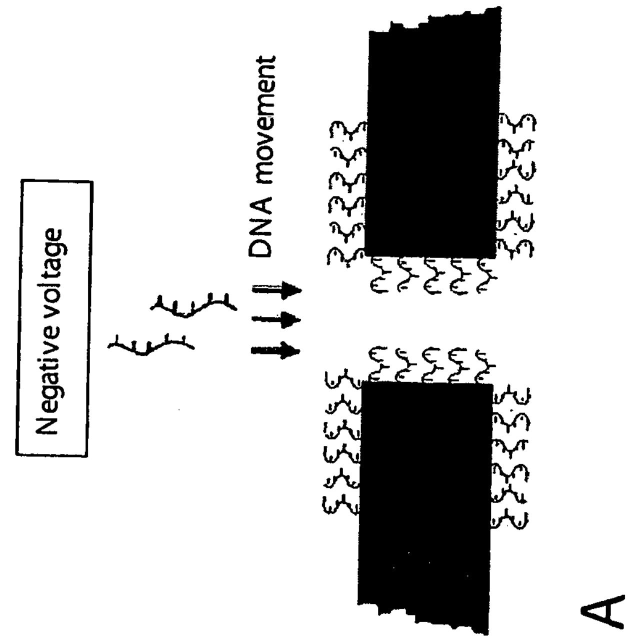


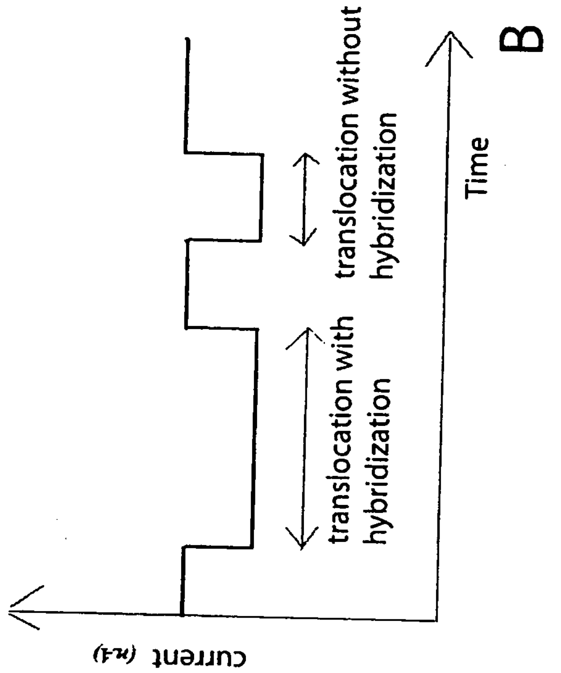


Fig. 5

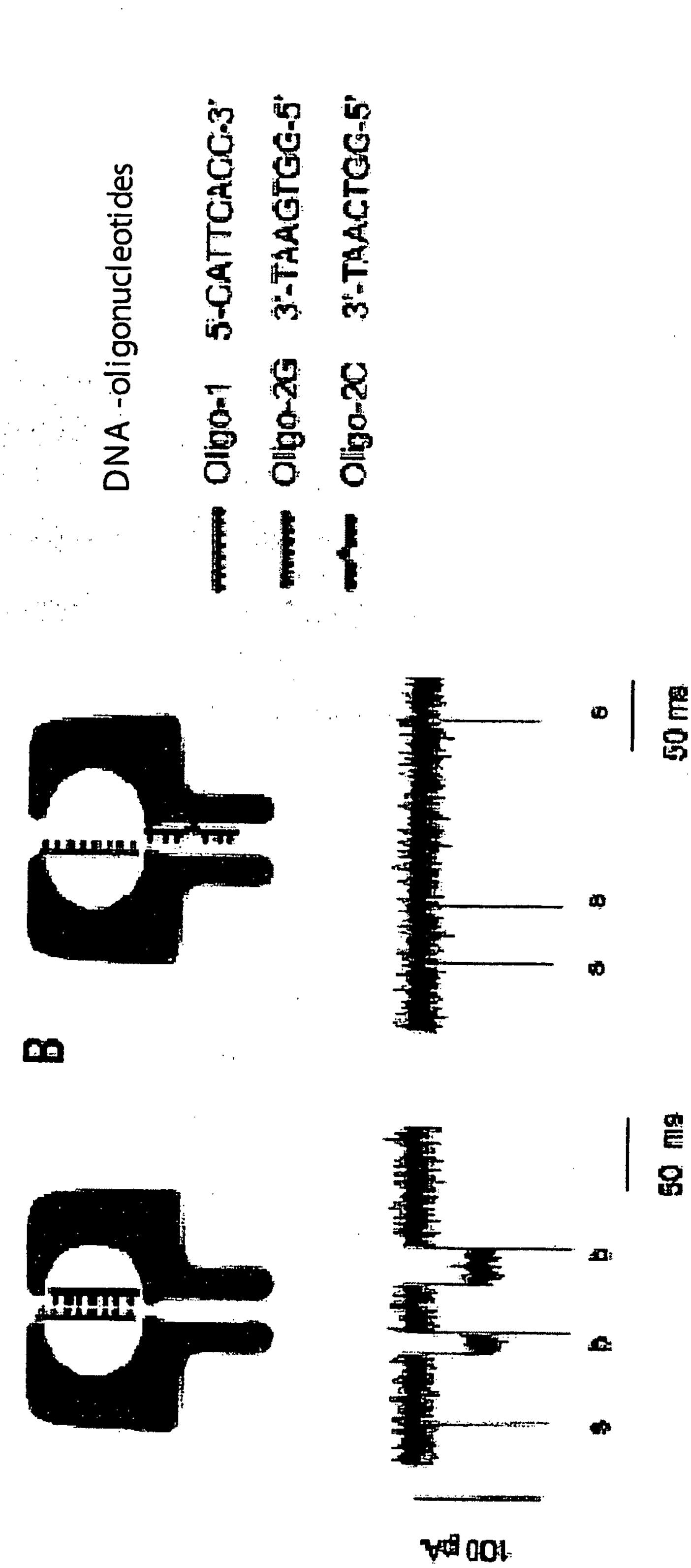
DNA -oligonucleotides
Oligo-1 5'-CATTCACC-3'
Oligo-2G 3'-TAAGTGG-5'
Oligo-2C 3'-TAACTGG-5'
B
50 ms
50 ms
100 pA

Fig. 6

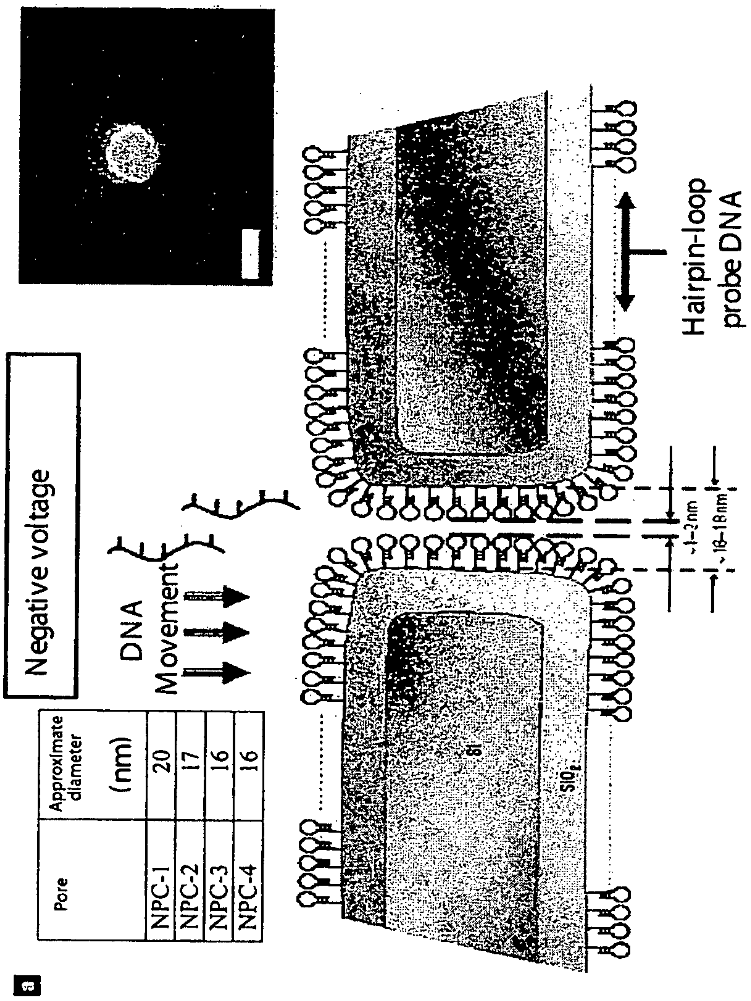
a
Pore
Approximate diameter (nm)
NPC-1 20
NPC-2 17
NPC-3 16
NPC-4 16
Negative voltage
DNA Movement
Si
SiO2
~1-2nm
~16-18nm
Hairpin-loop probe DNA

Fig. 7

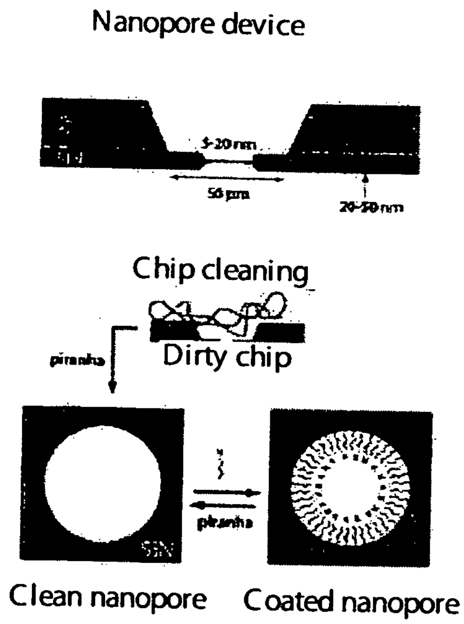
Nanopore device
5-20 nm
50 µm
20-50 nm
Chip cleaning
Dirty chip
piranha
SiN
piranha
Clean nanopore
Coated nanopore

Fig. 8

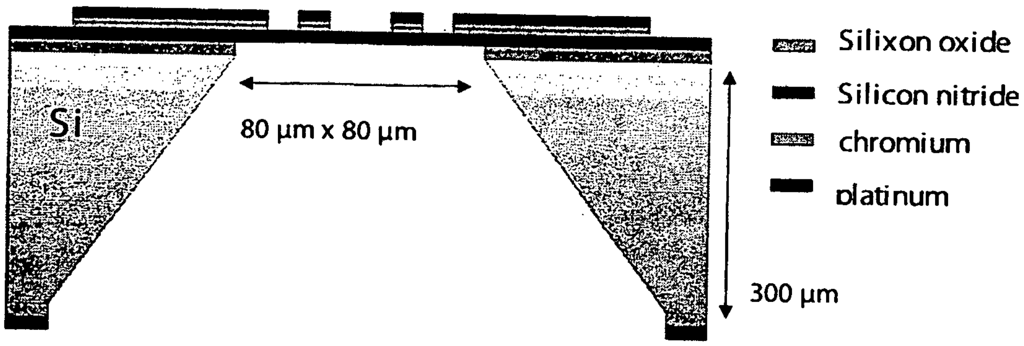
Si
80 µm x 80 µm
Silixon oxide
Silicon nitride
chromium
platinum
300 µm

Fig. 9

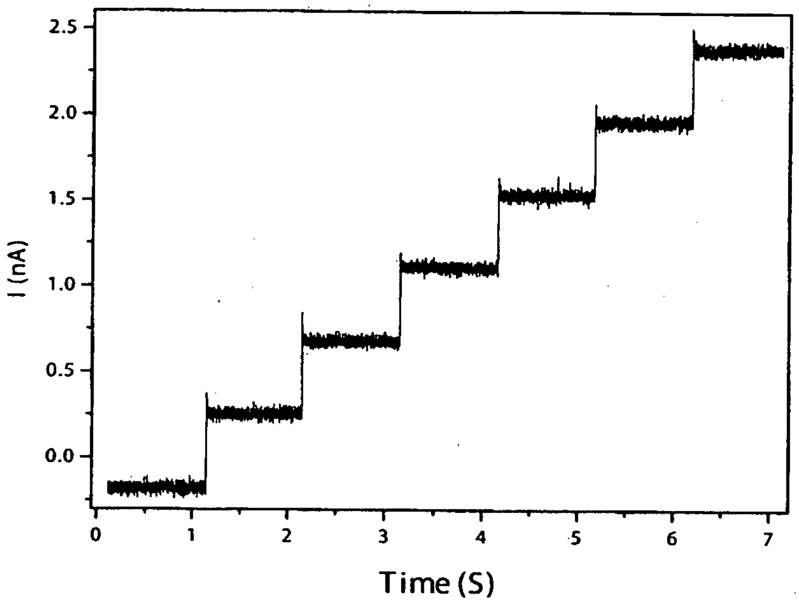
I (nA)
2.5
2.0
1.5
1.0
0.5
0.0
0
1
2
3
4
5
6
7
Time (S)

Fig. 10

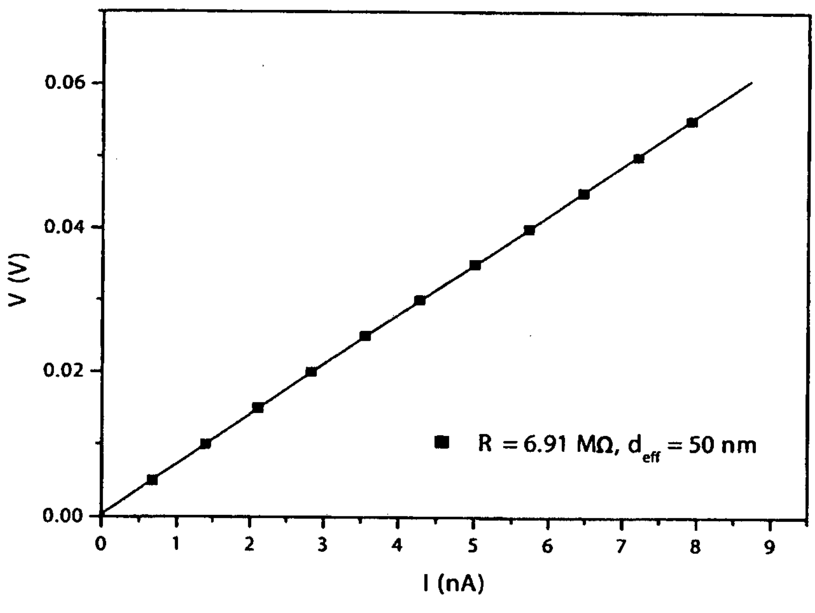
V (V)
0.06
0.04
0.02
0.00
0
1
2
3
4
5
6
7
8
9
I (nA)
R = 6.91 MΩ, d_eff = 50 nm

Fig. 11

Seq ID no:2 (seq. ID no : 1 grey)

aaattgagcc cgcagcctcc cgcttcgctc tctgctcctc ctgttcgaca gtcagccgca
tcttcttttg cgtcgccagc cgagccacat cgctcagaca ccatggggaa ggtgaaggtc
ggagtcaacg gatttggtcg tattgggcgc ctggtcacca gggctgcttt taactctggt
aaagtggata ttgttgccat caatgacccc ttcattgacc tcaactacat ggtttacatg
ttccaatatg attccaccca tggcaaattc catggcaccg tcaaggctga gaacgggaag
cttgtcatca atggaaatcc catcaccatc ttccaggagc gagatccctc caaaatcaag
tggggcgatg ctggcgctga gtacgtcgtg gagtccactg gcgtcttcac caccatggag
aaggctgggg ctcatttgca gggggagcc aaaagggtca tcatctctgc cccctctgct
gatgccccca tgttcgtcat gggtgtgaac catgagaagt atgacaacag cctcaagatc
atcagcaatg cctcctgcac caccaactgc ttagcacccc tggccaaggt catccatgac
aactttggta tcgtggaagg actcatgacc acagtccatg ccatcactgc cacccagaag
actgtggatg gcccctccgg gaaactgtgg cgtgatggcc gcggggctct ccagaacatc
atccctgcct ctactggcgc tgccaaggct gtgggcaagg tcatccctga gctgaacggg
aagctcactg gcatggcctt ccgtgtcccc actgccaacg tgtcagtggt ggacctgacc
tgccgtctag aaaaacctgc caaatatgat gacatcaaga aggtggtgaa gcaggcgtcg
gagggccccc tcaagggcat cctgggctac actgagcacc aggtggtctc ctctgacttc
aacagcgaca cccactcctc cacctttgac gctggggctg gcattgccct caacgaccac
tttgtcaagc tcatttcctg gtatgacaac gaatttggct acagcaacag ggtggtggac
ctcatggccc acatggcctc caaggagtaa gacccctgga ccaccagccc cagcaagagc
acaagaggaa gagagagacc ctcactgctg gggagtccct gccacactca gtcccccacc
acactgaatc tcccctcctc acagttgcca tgtagacccc ttgaagaggg gaggggccta
gggagccgca ccttgtcatg taccatcaat aaagtaccct gtgctcaacc

Fig. 12

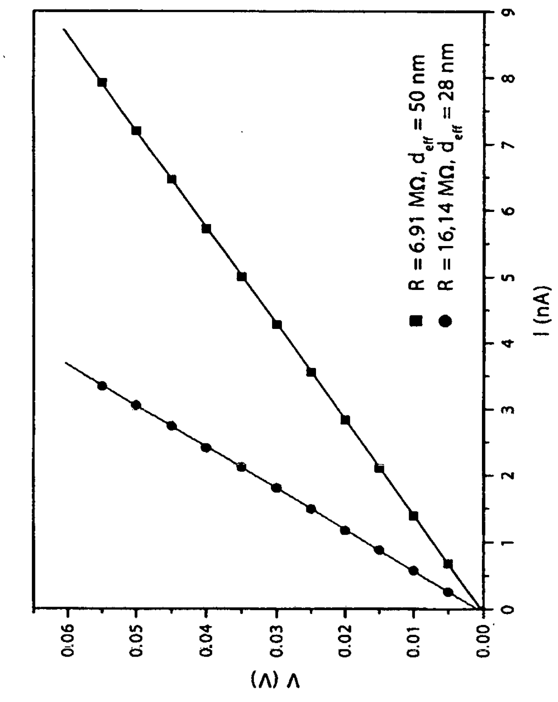
V (V)
0.00
0.01
0.02
0.03
0.04
0.05
0.06
I (nA)
0
1
2
3
4
5
6
7
8
9
R = 6.91 MΩ, $d_{eff}$ = 50 nm
R = 16,14 MΩ, $d_{eff}$ = 28 nm

Fig. 13

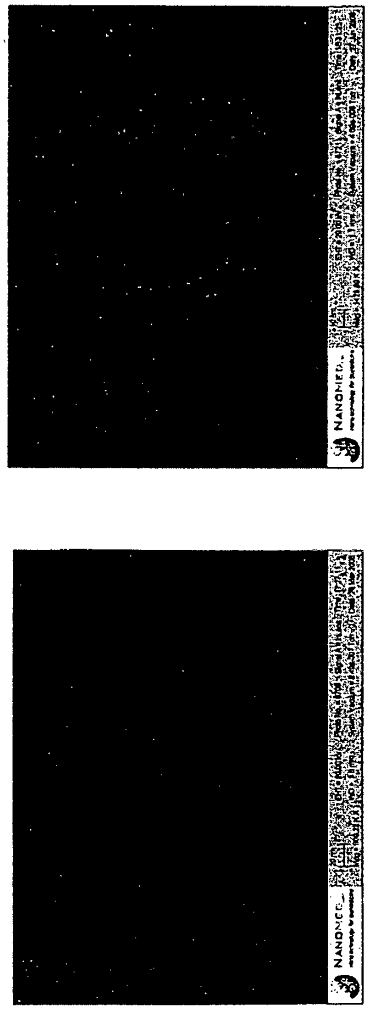

Fig. 14

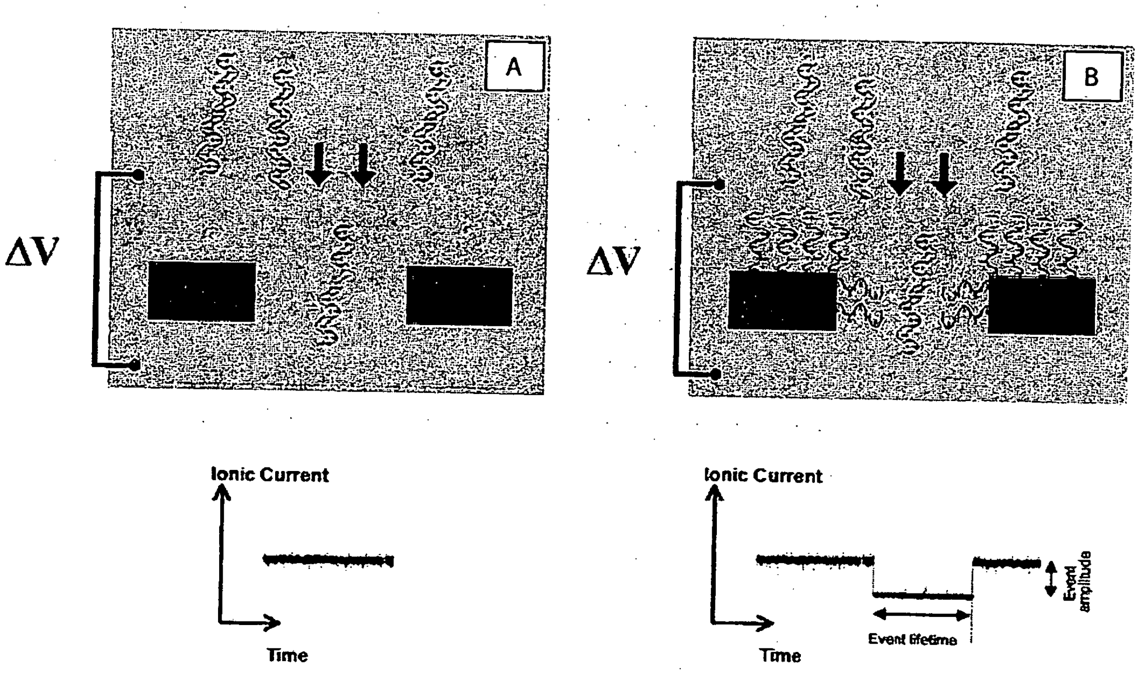
A
B
ΔV
ΔV
Ionic Current
Time
Ionic Current
Time
Event lifetime
Event amplitude

Fig 15

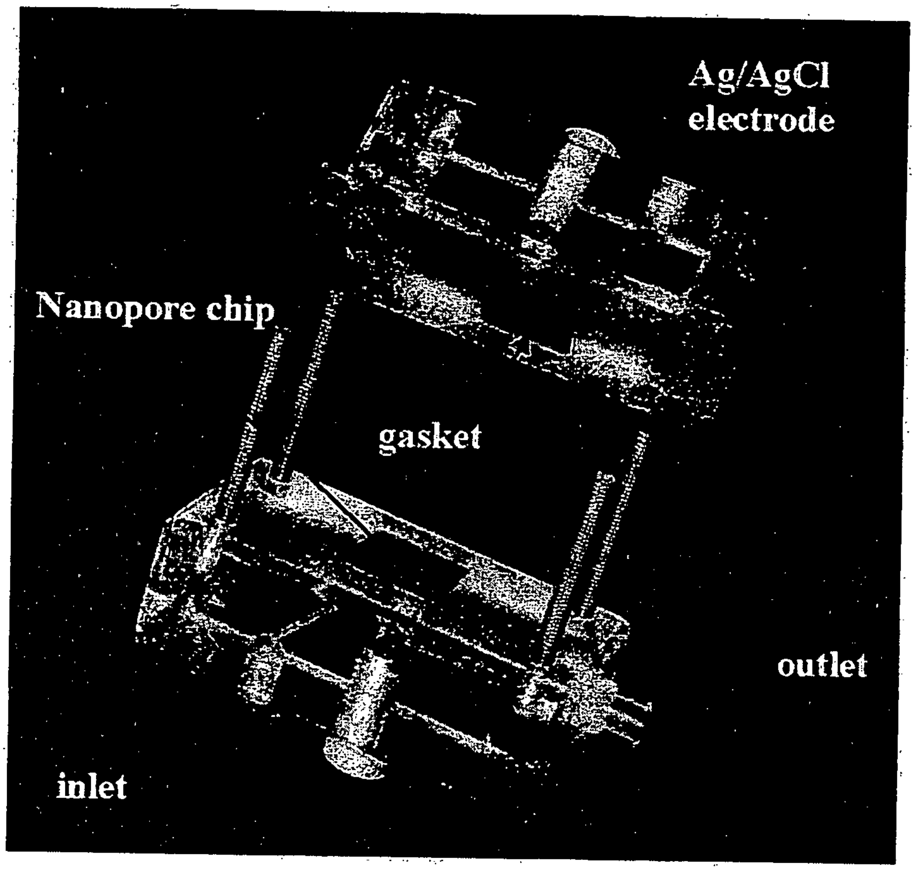
Ag/AgCl
electrode
Nanopore chip
gasket
outlet
inlet

Fig. 16

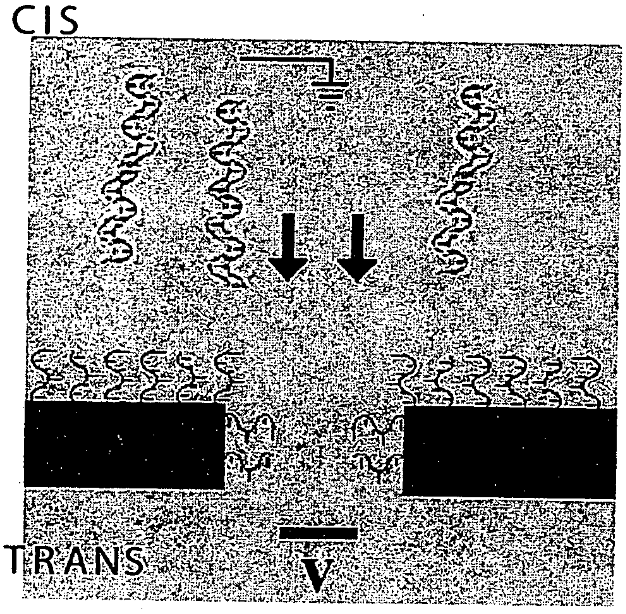
CIS
TRANS
V

Fig. 17

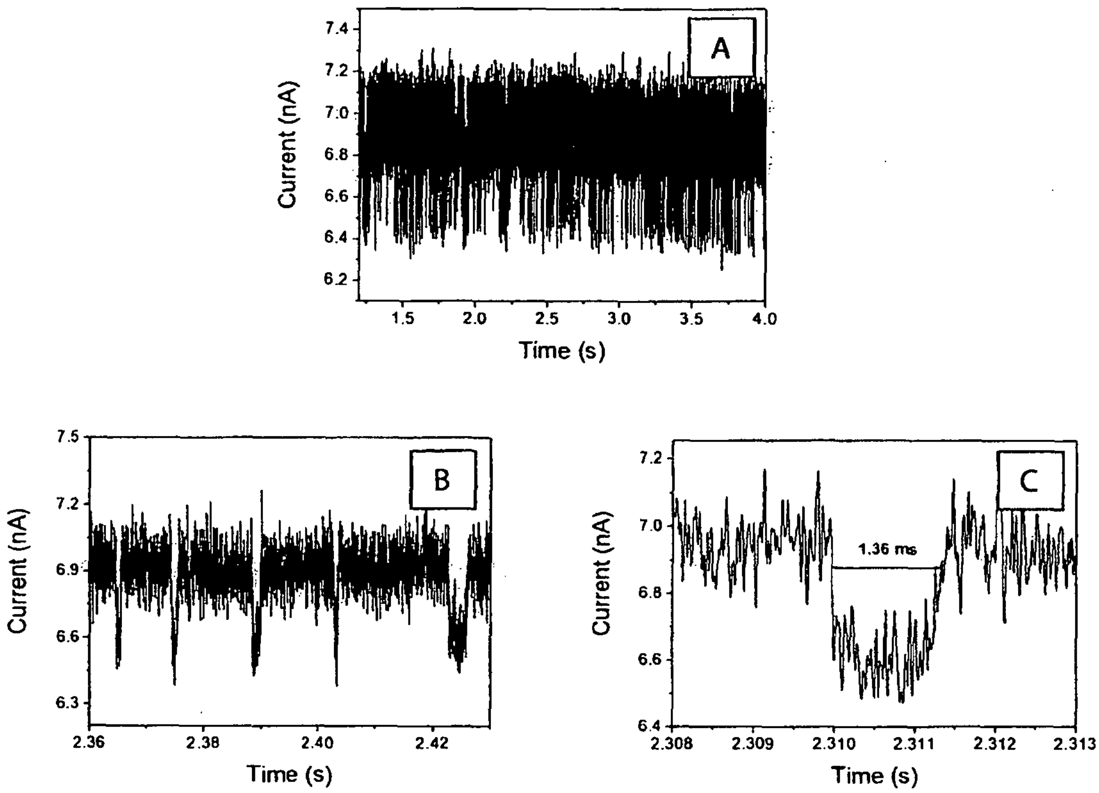
A
Current (nA)
7.4
7.2
7.0
6.8
6.6
6.4
6.2
1.5
2.0
2.5
3.0
3.5
4.0
Time (s)
B
Current (nA)
7.5
7.2
6.9
6.6
6.3
2.36
2.38
2.40
2.42
Time (s)
C
Current (nA)
1.36 ms
7.2
7.0
6.8
6.6
6.4
2.308
2.309
2.310
2.311
2.312
2.313
Time (s)

Fig. 18

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **A J STORM ; J H CHEN ; H W ZANDBERGEN ; C DEKKER.** Translocation of double-strand dna through a silicon oxide nanopore. *Physical Review,* 2005, vol. 71, 051903
- **S.HOWORKA ; S. CHELEY ; H.BAYLEY.** Sequence-specific detection of individual DNA strands using engineered nanopores. *Nature Biotechnology,* 2001, vol. 19, 636-639
- **S. M. IQBAL ; D. AKIN ; R.BASHIR.** *Nature Nanotechnology,* 2007, vol. 2, 243-248
- **M. WANUNU ; A. MELLER.** Chemically modified solid-state nanopores. *Nano Letters,* 2007, vol. 7 (6), 1580-1585
- **M. SCHENA.** Microarray biochip technology. Eaton Publishing, 2000
- **J. J. KASIANOWICZ ; E. BRANDIN ; D. BRANTON ; D.W. DEAMER.** *Procl. Natl. Acad. Sci. USA,* 1996, vol. 93, 13770-13773
- **WH. COULTER.** *Proceedings of the National Electronics Conference,* 1957, 1034-1042
- **S. HOWORKA ; S. CHELEY ; H. BAYLEY.** *Nature Biotechnology,* 2001, vol. 19, 636-639
- **S. WINTERS-HILT ; M. LANDRY ; M. AKESON ; M. TANASE ; I. AMIN ; A. COOMBS ; E. MORALES ; J. MILLET ; C. BARIBAULT ; S. SENDAMANGALAM.** *Cheminformatics BMC Bioinformatics,* 2006, vol. 7, S22
- **J. LI ; D. STEIN ; C. MCMULLAN ; D. BRANTON ; M. J. AZIZ ; J.A. GOLOVCHENKO.** *Nature,* 2001, vol. 412, 166-169
- **C. J. LO ; T. AREF ; A. BEZRYADIN.** *Nanotechnology,* vol. 17, 3264-3
- **MUSSI et al.** *Superlattices and Microstructures,* 10 October 2008, vol. 46 (1-2), 59-63
- **A J STORM ; J H CHEN ; H W ZANDBERGEN ; C DEKKER.** *Physical Review E,* 2005, vol. 71, 051903
- **M. WANUNU ; A. MELLER.** *Nano Letters,* 2007, vol. 7 (6), 1580-1585